(19) Europäisches Patentamt
European Patent Office
Office européen des brevets

(11) Veröffentlichungsnummer: **0 436 915 A2**

# (12) EUROPÄISCHE PATENTANMELDUNG

(21) Anmeldenummer: **90125207.2**

(22) Anmeldetag: **21.12.90**

(51) Int. Cl.5: **C07D 251/24**, //C07D251/22, C07D251/16

(30) Priorität: **10.01.90 DE 4000480**

(43) Veröffentlichungstag der Anmeldung: **17.07.91 Patentblatt 91/29**

(84) Benannte Vertragsstaaten: **CH DE FR GB LI**

(71) Anmelder: **BAYER AG**
**W-5090 Leverkusen 1 Bayerwerk(DE)**

(72) Erfinder: **Beck, Gunther, Dr.**
**Am Mittelberg 19**
**W-5090 Leverkusen 1(DE)**

## (54) Verfahren zur Herstellung von 2,4,6-Tricyano-1,3,5-triazin.

(57) Das erfindungsgemäße Verfahren zur Herstellung von 2,4,6-Tricyano-1,3,5-triazin besteht darin, 2,4,6-Trihalogen-1,3,5-triazine der Formel

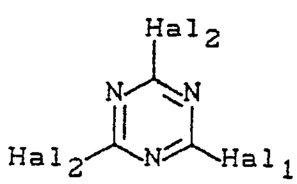

(I)

in der

$Hal_1$ und $Hal_2$ unabhängig voneinander für Chlor oder Fluor stehen,

mit Alkali- oder Erdalkalicyaniden umzusetzen.

EP 0 436 915 A2

## VERFAHREN ZUR HERSTELLUNG VON 2,4,6-TRICYANO-1,3,5-TRIAZIN

Die Erfindung betrifft ein neues Verfahren zur Herstellung von 2,4,6-Tricyano-1,3,5-triazin (Cyanurcyanid).

2,4,6-Tricyano-1,3,5-triazin ist eine interessante chemische Verbindung; sie bildet z.B. mit Tetrathiafulvalen elektrisch leitende Charge-Transfer-Komplexe (siehe Synthetic Metals 19, Seite 415 (1987)). Ferner reagiert die Verbindung beim Behandeln mit Methanol unter bestimmten Bedingungen unter Bildung von 2-Cyano-4,6-dimethoxy-1,3,5-triazin (Chem. Ber. 52, 659 (1919)), das seinerseits ein wertvolles Zwischenprodukt für die Herstellung hochwirksamer Herbizide darstellt (siehe EP-A 0 094 260).

Bislang steht aber noch kein Verfahren zur Verfügung, nach dem sich 2,4,6-Tricyano-1,3,5-triazin in größeren Mengen herstellen läßt. Das einzige bislang für die Herstellung der Verbindung beschriebene Verfahren besteht in einem Erhitzen des 2,4,6-Triscarbonamido-1,3,5-triazins mit Phosphorpentoxid und Sand (siehe Tetrahedron (1963) 19, Seite 161 bis 167, insbesondere Seite 162 und Seite 166).

Das als Ausgangsverbindung benötigte 2,4,6-Triscarbon-amido-1,3,5-triazin muß aber seinerseits in einem aufwendigen Vierstufenverfahren aus Oxalsäurediethylester hergestellt werden. Die Ausbeuten an reinem 2,4,6-Tri-cyano-1,3,5-triazin bezogen auf das Triscarbonamidotriazin betragen nur zwischen 12 bis 17 % der Theorie, d.h. das Verfahren kommt für eine Herstellung größerer Mengen des gewünschten Tricyanotriazins nicht in Betracht.

Es wurde jetzt gefunden, daß man das gewünschte 2,4,6-Tricyano-1,3,5-triazin auf einfache Weise ausgehend von wohlfeilen Ausgangsmaterialien durch Umsetzung von Alkali-oder Erdalkalicyaniden mit 2,4,6-Trihalogen-1,3,5-triazinen der Formel

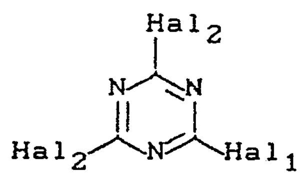

(I)

in der

Hal₁ und Hal₂ unabhängig voneinander für Chlor oder Fluor stehen

erhalten kann. Die Erfindung betrifft daher ein Verfahren zur Herstellung von 2,4,6-Tricyano-1,3,5-triazin, das dadurch gekennzeichnet ist, daß man 2,4,6-Trihalogen-1,3,5-triazine der Formel (I) mit Alkali- oder Erdalkalicyaniden umsetzt.

Es wurde gefunden, daß man 2,4,6-Tricyano-1,3,5-triazin aus 2,4,6-Trihalogen-triazinen der Formel (I), vorzugsweise aus 2,4,6-Trichlor-1,3,5-triazin (Cyanurchlorid) oder 2,4,6-Trifluor-1,3,5-triazin (Cyanurfluorid), in einer einfachen Einstufenreaktion in guten Ausbeuten und hoher Reinheit erhält, wenn man die Trihalogentriazine der Formel (I) mit Alkali- oder Erdalkalicyaniden in bestimmten Lösungsmitteln und unter milden Reaktionsbedingungen umsetzt und für die Umsetzung Alkali- und Erdalkalicyanide höchstens in einem geringen Überschuß über die stöchiometrisch erforderliche Menge anwendet. Durch die Parameter "milde Reaktionsbedingungen" und "höchstens geringer Überschuß an Alkali- oder Erdalkalicyaniden" wird erreicht, daß die Bildung unerwünschter Folgeprodukte, die aufgrund der hohen Reaktionsfähigkeit des Tricyanotriazins im Vordergrund steht, so weit unterbunden wird, daß das gewünschte Tricyanotriazin noch in guten Ausbeuten erhalten wird.

Die Erfindung betrifft daher im besonderen ein Verfahren zur Herstellung von 2,4,6-Tricyano-1,3,5-triazin, das dadurch gekennzeichnet ist, daß man 2,4,6-Trihalogen-1,3,5-triazine der Formel (I) in aliphatischen oder cyclischenEthern oder aliphatischen Nitrilen unter milden Reaktionsbedingungen mit höchstens einem geringen Überschuß über die stöchiometrisch erforderliche Menge an Alkali- oder Erdalkalicyaniden umsetzt.

Von den erfindungsgemäß als spezielle Lösungsmittel zu verwendenden aliphatischen und cyclischen Ethern und aliphatischen Nitrilen haben sich besonders Diethylenglykoldimethylether (Diglyme), 1,2-Dimethoxyethan, Tetrahydrofuran, Propionitril und Acetonitril bewährt. Besonders bevorzugt wird Acetonitril verwendet.

Als Alkali- und Erdalkalicyanide werden vorzugsweise die wohlfeilen Alkalicyanide Natrium- und Kaliumcyanid verwendet.

Für den Austausch der drei Halogenatome in den Cyanurhalogeniden der Formel (I) sind 1,5 Mol

Erdalkali- oder 3 Mol Alkalicyanid je Mol Trihalogen-triazin erforderlich.

Erfindungsgemäß werden die Trihalogentriazine der Formel (I) höchstens mit einem geringen Überschuß von maximal 20 Mol-%, bezogen auf die theoretisch für den vollständigen Umsatz der Halogenatome erforderliche Molmenge, umgesetzt. Es wurde aber gefunden, daß es durchaus vorteilhaft sein kann, mit einem Unterschuß an Cyaniden zu arbeiten; es bleibt dann zwar ein dem Unterschuß entsprechender Teil des Trihalogentriazins unumgesetzt zurück; da sich aber Tricyanotriazin auf einfache Weise vom z.B. unumgesetztem Trifluortriazin abtrennen läßt und dieses zurückgewonnene Trihalogen-triazin im nächsten Ansatz wiederverwendet werden kann, und durch die Verwendung eines Cyanidunterschusses die Folgeproduktbildung herabgesetzt wird, kann es durchaus sinnvoll sein, im erfindungsgemäßen Verfahren wesentlich weniger als die stöchiometrisch erforderliche Cyanidmenge zu verwenden. Ganz allgemein werden im erfindungsgemäßen Verfahren je Mol Trihalogentriazin 0,3 bis 3,6 Äquivalente Alkali- oder Erdalkalicyanid eingesetzt.

Die erfindungsgemäße Umsetzung der Trihalogentriazine der Formel (I) mit den Alkali- oder Erdalkalicyaniden verläuft zwar, wie man bei der gaschromatographischen Verfolgung der Umsetzung feststellen kann, über die Zwischenstufen des Monocyano-dihalogeno- und Dicyanomonohalogeno-1,3,5-triazins; trotzdem entsteht auch bei Verwendung eines Cyanid-Unterschusses im wesentlichen nur das 2,4,6-Tricyano-1,3,5-triazin, wenn man die Reaktion bis zu ihrem Ende durchführt.

Die erfindungsgemäße Umsetzung der Trihalogentriazine mit den Erdalkali- und Alkalicyaniden läßt sich durch folgende Reaktionsgleichung beschreiben:

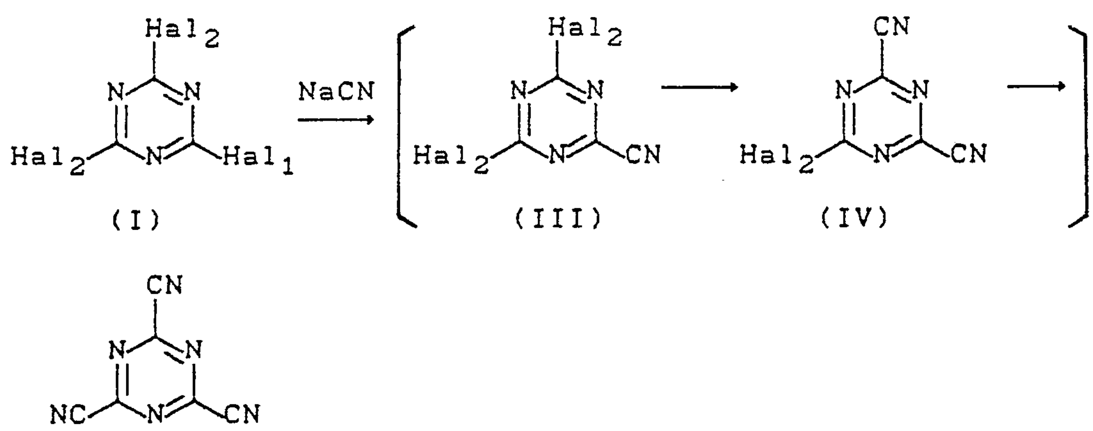


(I) (III) (IV)

(II)

Monocyanodihalogen-1,3,5-triazin (III) und Dicyanomonohalogen-1,3,5-triazin (IV) sind vor allem dann faßbar und durch Gaschromatographie nachweisbar, wenn man die erfindungsgemäße Umsetzung mit einem Unterschuß an Cyaniden durchführt und vorzeitig abbricht. Im Gaschromatogramm liegen die Retentionszeiten von (III) und (IV) zwischen den Retentionszeiten von (I) und (II).

Die erfindungsgemäße Umsetzung wird unter milden Bedingungen, d.h. bei Temperaturen von -50 bis +80 °C, bevorzugt bei -40 bis +50 °C und besonders bevorzugt bei -30 bis +30 °C vorgenommen. Die Temperatur richtet sich nach dem verwendeten Trihalogen-triazin; das äußerst reaktionsfähige Cyanurfluorid erfordert die niedrigeren, das etwas reaktionsträgere Cyanurchlorid die etwas höheren Reaktionstemperaturen. Der Verlauf der Umsetzung läßt sich gaschromatographisch verfolgen. Die Reaktion ist beendet, sobald sich die Zusammensetzung des Reaktionsgemisches gemäß Gaschromatogramm nicht mehr verändert. Die Reaktionszeiten bis zu diesem Reaktionsende schwanken je nach Trihalogentriazin, Ansatzgröße und Reaktionstemperaturen zwischen 1 und 200 Stunden.

Die erfindungsgemäße Umsetzung wird vorzugsweise so ausgeführt, daß man die Lösung des Trihalogentriazins im betreffenden wasserfreien Lösungsmittel bei Temperaturen von -30 bis +30 °C unter Rühren entweder portionsweise oder unmittelbar mit der gesamten, zur Umsetzung vorgesehenen Cyanidmenge versetzt und die Temperatur des Reaktionsgemisches langsam unter Rühren auf die vorgesehene Endtemperatur ansteigen läßt.

Die Aufarbeitung des Reaktionsgemisches erfolgt bevorzugt in der Weise, daß man die Alkali- und Erdalkalihalogenide (Chloride bzw. Fluoride) mechanisch, z.B. durch Filtration abtrennt, das Filtrat im Vakuum vom Lösungsmittel befreit und das Tricyanotriazin unmittelbar aus dem Rückstand durch Sublimation (z.B. bei 100 bis 110 °C/0,1 mbar) oder durch Umkristallisieren, z.B. aus Benzol, isoliert. Als vorteilhafter hat es sich jedoch erwiesen, den Rückstand zunächst mit einem Lösungsmittel zu behandeln, in dem das Tricyanotriazin löslich ist, die unerwünschten Folgeprodukte jedoch praktisch unlöslich sind. Derartige Lösungsmittel sind z.B. Di- oder Trichlormethan. Nach Abtrennen der in dem Lösungsmittel unlöslichen Stoffe und Einengen des Filtrates im Vakuum wird ein bereits praktisch reines Tricyanotriazin erhalten. Dieses kann, falls gewünscht, durch Umkristal-lisieren und/oder Sublimieren noch weiter gereinigt werden. Da z.B. Cyanurchlorid in Benzol deutlich leichter löslich ist als das Tricyanotriazin, läßt sich nicht umgesetztes Cyanurchlorid durch Umkristallisieren aus Benzol oder chromatographisch abtrennen.

## Beispiele

### Beispiel 1

Eine im Eisbad auf 0 bis 5 °C gekühlte Lösung von 184,4 g (1 Mol) Cyanurchlorid in 2500 ml wasserfreiem Acetonitril wird unter Rühren und unter Feuchtigkeitsaus-schluß mit 152 g (3,1 Mol) Natriumcyanid versetzt. Das Reaktionsgemisch wird etwa 70 Stunden kräftig gerührt, davon zunächst etwa 12 Stunden unter Eisbadkühlung, danach bei Raumtemperatur.

Die Aufarbeitung des Reaktionsgemisches wird unter Ausschluß von Luftfeuchtigkeit wie folgt durchgeführt: Der Rückstand wird abfiltriert und im Vakuum im Rotationsverdampfer bei Raumtemperatur zur Trockne eingeengt. Der ölige Rückstand wird bei Raumtemperatur kräftig mit wasserfreiem Dichlormethan (etwa 1 l) verrührt. Nach dem Abfiltrieren vom Ungelösten wird das Filtrat im Rotationsverdampfer zunächst bei Raumtemperatur, abschließend bei 30 °C zur Trockne eingeengt. Das in Form eines trockenen, kristallinen bräunlichen Pulvers zurückbleibende rohe 2,4,6-Tricyano-1,3,5-triazin (109 g = 70 % der Theorie) wird durch Sublimation bei 100 bis 110 °C/0,1 mbar in Form rein weißer Kristalle erhalten.
Ausbeute: 92,7 g (= 59,4 % der Theorie).
IR (KBr) in $cm^{-1}$: 2276, 2255, 1653, 1555, 1515, 1339, 939, 822.

Die Lagen der Absorptionsbanden stimmen im wesentlichen mit den Angaben der Literatur (in Nujol gemessen; Tetrahedron 19, 161-167 (1973)) überein.

### Beispiel 2

Die Lösung von 36,9 g (0,2 Mol) Cyanurchlorid in 750 ml wasserfreiem Acetonitril wurde unter den in Beispiel 1 beschriebenen Reaktionsbedingungen mit 32,34 g (0,66 Mol) Natriumcyanid umgesetzt. Die gaschromatographische Analyse des Reaktionsgemisches ergab nach 45 Stunden, daß kein Cyanurchlorid mehr vorhanden war. Das Reaktionsgemisch wurde wie in Beispiel 1 beschrieben aufgearbeitet.
Ausbeute an reinem 2,4,6-Tricyano-1,3,5-triazin: 14,0 g (= 44,9 % der Theorie).

### Beispiel 3

Die Lösung von 12,0 g (0,065 Mol) Cyanurchlorid in 250 ml wasserfreiem Acetonitril wird bei Raumtemperatur unter kräftigem Rühren und unter Feuchtigkeitsausschluß mit 13,97 g (0,215 Mol) Kaliumcyanid versetzt; dabei erwärmt sich das Reaktionsgemisch zunächst auf 35-40 °C.

Die gaschromatographische Analyse des Reaktionsgemisches ergab nach 24 Stunden ein Verhältnis von Cyanurchlorid zu 2,4,6-Tricyano-1,3,5-triazin wie etwa 1:99. Nach weiterem 24-stündigem Rühren war kein Cyanurchlorid mehr nachweisbar.

Das Reaktionsgemisch wurde wie in Beispiel 1 beschrieben aufgearbeitet.
Ausbeute an reinem 2,4,6-Tricyano-1,3,5-triazin: 4,5 g (= 44,4 % der Theorie).

### Beispiel 4

Eine auf -35 °C abgekühlte Lösung von 27,0 g (0,2 Mol) Cyanurfluorid in 500 ml wasserfreiem Acetonitril wird unter Rühren und unter Feuchtigkeitsausschluß mit 4,9 g (0,1 Mol) Natriumcyanid versetzt. Nach dem Entfernen des Kältebades steigt die Temperatur des Reaktionsgemisches im Verlauf von etwa 1 Stunde auf 0 °C an. Mittels Eisbad wird die Temperatur des Reaktionsgemisches etwa 8 Stunden auf 0 °C gehalten. Anschließend läßt man die Temperatur des Reaktionsgemisches im Verlauf weiterer 13 Stunden allmählich auf 13 °C ansteigen.

Das Reaktionsgemisch wird anschließend im Rotationsverdampfer zur Trockne eingeengt und der Rückstand mit Dichlormethan (etwa 250 ml) kräftig verrührt. Nach dem Abfiltrieren von Ungelöstem wird

das Filtrat bei Raumtemperatur im Rotationsverdampfer eingeengt; durch Anlegen von Hochvakuum werden letzte Reste von Dichlormethan und Cyanurfluorid entfernt.
Ausbeute an rohem 2,4,6-Tricyano-1,3,5-triazin: 4,25 g (= 81,7 % der Theorie bezogen auf eingesetztes Natriumcyanid); das Rohprodukt wird durch Sublimation bei 120° C/0,1 mbar gereinigt;
Ausbeute an reinem 2,4,6-Tricyano-1,3,5-triazin: 4,11 g (= 79 % der Theorie, bezogen auf eingesetztes Natriumcyanid).

Die gaschromatographische und die daran anschließende massenspektroskopische Analyse (GC-MS-Analyse) des Reaktionsgemisches ergab, daß die beiden Zwischenstufen 2-Cyano-4,6-difluor-1,3,5-triazin und 2,4-Dicyano-6-fluor-1,3,5-triazin zu Beginn der Reaktion, d.h. nachdem das Reaktionsgemisch etwa 2 Stunden bei 0° C gerührt worden war, im Reaktionsgemisch enthalten waren.

Beispiel 5

Eine im Eisbad auf 1 bis 3° C abgekühlte Lösung von 27,66 g (0,15 Mol) Cyanurchlorid in 500 ml wasserfreiem Acetonitril wird unter Rühren und unter Feuchtigkeitsausschluß mit 7,35 g (0,15 Mol) Natriumcyanid versetzt. Die Reaktionsmischung wird zunächst etwa 10 Stunden im Eisbad, anschließend etwa 110 Stunden bei etwa 15° C kräftig gerührt. Die Zusammensetzung des Reaktionsgemisches wurde nach 24 Stunden, nach 48 Stunden und nach 120 Stunden mittels GC-MS bestimmt; dabei ergaben sich folgende Gehalte an Cyanurchlorid, Tricyanotriazin, Dichlormonocyanotriazin und Monochlordicyanotriazin.

| Zeit [h] | 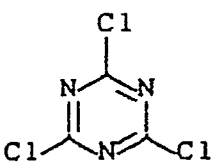 | 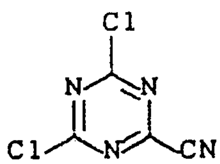 | 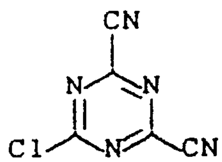 | 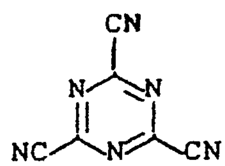 |
|---|---|---|---|---|
| 24 | 61,1 % | 4,4 % | 1,1 % | 32,9 % |
| 48 | 60,5 % | 3,8 % | 0 % | 35,7 % |
| 120 | 62,3 % | 3,8 % | 0 % | 33,9 % |

Das Reaktionsgemisch wurde wie in Beispiel 1 beschrieben aufgearbeitet.
Ausbeute an reinem Tricyanotriazin nach Umkristallisieren aus Benzol: 41 % der Theorie, bezogen auf eingesetztes Natriumcyanid.

Beispiel 6

Es wurde wie in Beispiel 1 beschrieben gearbeitet mit dem einzigen Unterschied, daß das Reaktionsgemisch 140 Stunden bei 0 bis 5° C gerührt wurde.
Ausbeute an sublimiertem 2,4,6-Tricyano-1,3,5-triazin: 89,9 g (= 57,6 % der Theorie).

Beispiel 7

Die Lösung von 18,44 g (0,1 Mol) Cyanurchlorid in 400 ml wasserfreiem Tetrahydrofuran wird mit 15,5 g (0,316 Mol) Natriumcyanid versetzt und 120 Stunden bei Raumtemperatur unter Feuchtigkeitsausschluß kräftig gerührt. Gemäß GC-Analyse ist dann kein Cyanurchlorid mehr im Reaktionsgemisch vorhanden.

Das Reaktionsgemisch wird wie in Beispiel 1 beschrieben aufgearbeitet.
Ausbeute an reinem sublimiertem 2,4,6-Tricyano-1,3,5-triazin: 7,09 g (= 45,4 % der Theorie).

Beispiel 8

Man verfährt analog Beispiel 1 mit dem einzigen Unterschied, daß anstatt 152 g (3,1 Mol) Natriumcyanid 142 g (2,9 Mol) Natriumcyanid eingesetzt werden. Die Reaktionszeit beträgt etwa 120 Stunden.

Reinstausbeute nach Sublimation: 94,1 g (= 60,3 % der Theorie) an 2,4,6-Tricyano-1,3,5-triazin.

Vergleichbare Ausbeuten an 2,4,6-Tricyano-1,3,5-triazin werden erzielt, wenn man als Lösungsmittel statt Acetonitril 1,2-Dimethoxyethan verwendet.

## Patentansprüche

1. Verfahren zur Herstellung von 2,4,6-Tricyano-1,3,5-triazin, dadurch gekennzeichnet, daß man 2,4,6-Trihalogen-1,3,5-triazine der Formel

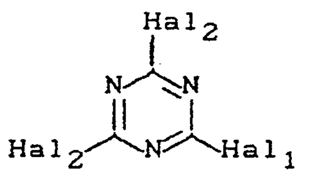

(I)

in der
$Hal_1$ und $Hal_2$ unabhängig voneinander für Chlor oder Fluor stehen,
mit Alkali- oder Erdalkalicyaniden umsetzt.

2. Verfahren nach Anspruch 1, dadurch gekennzeichnet, daß man 2,4,6-Trihalogen-1,3,5-triazine der Formel (I) in aliphatischen oder cyclischen Ethern oder aliphatischen Nitrilen unter milden Reaktionsbedingungen mit höchstens einem geringen Überschuß über die stöchiometrisch erforderliche Menge an Alkali- oder Erdalkalicyaniden umsetzt.

3. Verfahren nach Anspruch 2, dadurch gekennzeichnet, daß man je Mol 2,4,6-Trihalogen-1,3,5-triazin 0,3 bis 3,6 Äquivalente Alkali- oder Erdalkalicyanid verwendet.

4. Verfahren nach Ansprüchen 2 oder 3, dadurch gekennzeichnet, daß man die Umsetzung bei Temperaturen von -50 bis +80 °C vornimmt.

5. Verfahren nach Ansprüchen 2 oder 3, dadurch gekennzeichnet, daß man die Umsetzung bei Temperaturen von -40 bis +50 °C vornimmt.

6. Verfahren nach Ansprüchen 2 oder 3, dadurch gekennzeichnet, daß man die Umsetzung bei Temperaturen von -30 bis +30 °C vornimmt.